# EUROPÄISCHE PATENTSCHRIFT

(11) **EP 2 777 662 B1**
(45) Veröffentlichungstag und Bekanntmachung des Hinweises auf die Patenterteilung: **08.07.2015**
(21) Anmeldenummer: 14000368.2
(22) Anmeldetag: 31.01.2014
(51) Int. Cl.: A61F 13/00, A61F 13/36, D04H 1/46, D04H 3/105, D04H 3/147, D04H 5/02, D04H 11/08

(54) **VERWENDUNG EINES VELOURSNADELVLIESSTOFFS**
USE OF A VELVET FIBREBONDED FABRIC SUBSTANCE
UTILISATION D'UN NON-TISSÉ AIGUILLETÉ EN VELOURS

(30) Priorität: 11.03.2013 DE 102013004089; 26.08.2013 DE 102013014025
(43) Veröffentlichungstag der Anmeldung: 17.09.2014
(73) Patentinhaber: Carl Freudenberg KG, 69469 Weinheim (DE)
(72) Erfinder: Mass, Valentina, 69488 Birkenau (DE); Lange, Birger, 64295 Darmstadt (DE); Schoepping, Gerhard, 69469 Weinheim (DE); Schlesselmann, Bernd, 69469 Weinheim (DE)

(56) Entgegenhaltungen:
- EP-A2- 0 864 289
- WO-A1-2010/085831
- US-A- 3 561 441
- US-A1- 2006 286 343
- US-A1- 2009 117 804
- US-B1- 8 152 929

## Beschreibung

### Technisches Gebiet

Die Erfindung betrifft eine Verwendung eines Veloursnadelvliesstoffs.

### Stand der Technik

Aus der EP 2 365 794 B1 ist eine Wundreinigungseinrichtung bekannt geworden, welche von einer Trägerschicht abstehende Fäden mit frei auskragenden Enden aufweist. Diese Fäden weisen schräg zu ihrer Längserstreckung verlaufende Enden bzw. Endflächen auf.

Hierbei ist nachteilig, dass offene oder gebrochene Enden Hautirritationen auf der menschlichen Haut verursachen können. Dies ist belegt durch den Abschlussbericht des Forschungsvorhabens AiF-Nr. 14 655 N, erhältlich am Institut für Textil- und Verfahrenstechnik, Denkendorf.

Vor diesem Hintergrund besteht ein Bedarf nach Wundreinigungseinrichtungen, welche möglichst wenig Hautirritationen erzeugen und dennoch Wunden gründlich reinigen können.

Aus der WO 2009/039 914 A1 ist bereits ein Veloursnadelviiesstoff bekannt, welcher im Automobilbereich, in der Schifffahrt oder in Bahnen verwendet werden kann.

In der Medizintechnik finden Vliesstoffe verschiedenster Art Verwendung, um Wunden zu versorgen oder zu behandeln. Insbesondere besteht ein Bedarf nach Wundreinigungsprodukten, welche Gewebe und Körperflüssigkeiten von Wunden in sich oder an sich aufnehmen. Dabei besteht ein besonderer Bedarf nach Wundreinigungsprodukten, welche aufgrund ihrer Struktur Wundgewebepartikel in sich oder an sich binden können.

Des Weiteren wird oft gewünscht, dass auch Reste von Verbandsmaterialien, die bei der Wundversorgung in einer Wunde zurückbleiben können, ebenfalls entfernt werden.

### Darstellung der Erfindung

Der Erfindung liegt daher die Aufgabe zugrunde, ein Wundreinigungsprodukt anzugeben, mit welchem eine Wunde und die diese umgebende Haut eines menschlichen oder tierischen Körpers schonend gereinigt werden können.

Die vorliegende Erfindung löst die zuvor genannte Aufgabe durch die Merkmale des Patentanspruchs 1.

Erfindungsgemäß ist erkannt worden, dass ein Veloursnadelvliesstoff überraschend gut als wundreinigendes Mittel für unterschiedliche Haut- und Gewebeschichten geeignet ist. Konkret ist erkannt worden, dass die Schlaufen eines Veloursnadelvliesstoffs Verkrustungen, abgestorbene Zellen oder Sekrete im Veloursnadelvliesstoff zurückhalten können. Es können problemlos insbesondere Nekrosen und Detritus entfernt werden, um eine Wundheilung zu ermöglichen. Anderenfalls würden die Wunden stagnieren. Des Weiteren können Biofilme und Bakterien derart entfernt werden, dass die mikrobielle Belastung reduziert ist. Die Schlaufen des Veloursnadelvliesstoffs reinigen Wunden überraschend schonend, so dass diese nicht gereizt werden.

Erfindungsgemäß werden Schlaufen und gerade keine abstehenden Fäden mit frei auskragenden Enden zur Reinigung von Wunden genutzt. Durch eine Vielzahl von Schlaufen und bevorzugt keine abstehenden Fäden mit frei auskragenden Enden, werden Hautirritationen nahezu vollständig vermieden.

Erfindungsgemäß wird daher gerade kein Rasierklingeneffekt wie in der EP 2 365 794 B1 genutzt, der durch schräge Endflächen frei auskragender Fäden bewirkt wird, sondern ein flächiges Anlegen von Schlaufen auf der Haut. Durch das flächige Anlegen werden Partikel in den Schlaufen aufgenommen und von diesen mitgenommen.

Folglich ist die eingangs genannte Aufgabe gelöst.

Vor diesem Hintergrund ist auch denkbar, dass der Veloursnadelvliesstoff derart ausgerüstet oder ausgestaltet ist, dass er Wundexsudate problemlos absorbieren kann.

Der Veloursnadelvilesstoff könnte durch Ablage eines zumindest teilweise vorverfestigten Vliesstoffs auf einer Stichunterlage und durch Nadeln des Vliesstoffs auf dieser Stichunterlage hergestellt sein. Durch das Ablegen eines zumindest teilweise vorverfestigten Vliesstoffs auf einer Stichunterlage bilden sich beim Nadeln Schlaufen. Die Schlaufen halten Verkrustungen, abgestorbene Zellen oder Sekrete im Material des Veloursnadelvliesstoffs zurück.

Der Veloursnadelvliesstoff könnte nur eine einzige Lage aufweisen, von welcher Schlaufen abragen. Hierbei wird vorteilhaft, das Gewicht reduziert und der Fertigungsprozess vereinfacht. Die reinigenden Funktionen des Veloursnadelvliesstoffs werden durch Verwendung nur einer Lage nicht negativ beeinträchtigt. Des Weiteren wird der Veloursnadelvliesstoff flexibler als bei Verwendung zweier Lagen.

Der Veloursnadelvliesstoff könnte eine erste Lage und eine zweite Lage aufweisen, wobei die erste Lage erste Fasern umfasst, die in Schlaufen gezwungen von der ersten Lage abragen, und wobei die zweite Lage zweite Fasern umfasst, welche mit der ersten Lage zumindest bereichsweise verbunden sind. Durch Verwendung zweier Lagen wird die mechanische Stabilität erhöht.

Vor diesem Hintergrund ist konkret denkbar, dass die zweiten Fasern der zweiten Lage ebenfalls durch Nadeln in die Fasern der ersten Lage getrieben und mit diesen verschlungen werden. Die zweite Lage dient insoweit als Decklage, welche auf der Seite der ersten Lage anliegt, welche den Schlaufen abgewandt ist. Durch die Verwendung einer zweiten Lage wird die erste Lage stabilisiert.

Vor diesem Hintergrund könnten auch die zweiten Fasern in Schlaufen gezwungen sein. Die zweiten Fasern der zweiten Lage werden durch die Nadeln in Schlaufen gezwungen und in die erste Lage getrieben, um dort mit den ersten Fasern verschlungen zu werden.

Es ist denkbar, dass auch die zweiten Fasern gemeinsam mit den ersten Fasern als Schlaufen einer Wunde oder der Haut, welche die Wunde umgibt, zugewandt sind.

Der Veloursnadelvliesstoff könnte erste und/ oder zweite Fasern umfassen, die als Mehrkomponentenfasern ausgebildet sind, welche das erste und das zweite Polymer aufweisen. Da das erste Polymer bei einer höheren Temperatur schmilzt als das zweite Polymer, kann das zweite Polymer zur Bindung verwendet werden. Die Verwendung von Mehrkomponentenfasern erlaubt, dass ein Teil einer Faser angeschmolzen wird, wobei der andere Teil fest verbleibt. Durch diese Maßnahme kann eine Mehrkomponentenfaser sowohl als strukturbildende Faser als auch als bindende Faser verwendet werden.

Vor diesem Hintergrund ist denkbar, Bikomponentenfasern zu verwenden. Als Bikomponentenfasern könnten Kern-Mantel-Fasern, Side-by-side-Fasern, Island-in-the-sea-Fasern und/ oder PIE-Fasern verwendet werden.

Bikomponentenfasern mit kuchenstückförmiger Geometrie oder Fasern oder Filamente aus Spinndüsen mit knotenförmiger Kapillar-Geometrie führen zu einer besonders guten Verankerung der Fasern oder Filamente im Veloursnadelvliesstoff. Hierdurch erhöhen sich die Zugfestigkeit und die Abriebfestigkeit des Veloursnadelvliesstoffs.

Durch den Anteil an Mehrkomponentenfasern oder Bikomponentenfasern lässt sich in einfacher Weise die Steifigkeit des Veloursnadelvliesstoffs einstellen. Je höher der Anteil an diesen Fasern ist, desto steifer wird das Material des Veloursnadelvliesstoffs.

Sofern keine Mehrkomponentenfasern, sondern Mischungen aus Monokomponentenfasern verwendet werden, kann die Steifigkeit des Veloursnadelvliesstoffs durch den Anteil an Monokomponentenfasern mit niedrigschmelzender Komponente eingestellt werden. Je mehr Fasern mit niedrigschmelzender Komponente eingesetzt werden, desto steifer kann der Veloursnadelvliesstoff werden.

Des Weiteren können Mehrkomponentenfasern durch Nadelvorgänge in Elementarfasern aufgesplittet werden. Hierdurch kann die Fasergeometrie derart verändert werden, dass die Verankerung der entstandenen Elementarfasern im Veloursnadelviiesstoff besonders hoch ist.

Die Fasergeometrie nimmt des Weiteren erheblichen Einfluss auf das Vermögen des Veloursnadelvliesstoffs, Schmutz an seine Fasern anzubinden.

Der Veloursnadelviiesstoff könnte als erste und/ oder zweite Fasern strukturbildende Fasern mit dem ersten Polymer und als erste und/oder zweite Fasern bindende Fasern mit dem zweiten Polymer umfassen. Vor diesem Hintergrund ist konkret denkbar, dass einige Fasern ausschließlich aus dem ersten Polymer und andere Fasern ausschließlich aus dem zweiten Polymer gebildet sind. In einem solchen Fall würden die einen Fasern eine Lage strukturell festigen und als strukturbildende Fasern fungieren, wobei die anderen Fasern im angeschmolzenen Zustand nach Art eines Bindemittels die strukturbildenden Fasern miteinander verbinden.

Zumindest ein Teil der ersten und/ oder zweiten Fasern des Veloursnadelvliesstoffs könnte als Filamente ausgebildet sein. Filamente sind so genannte Endlosfasern, die durch Spinndüsen gefertigt werden.
Endlosfasern führen zu einem sehr geringen Faserverlust, da diese nur schwer aus einem Verbund von Fasern herausfallen können. Ein geringer Faserverlust kann auch dadurch bewirkt werden, dass bindende Fasern eingesetzt werden. Durch diese Maßnahmen existieren nur wenig freie Fasern, die aus einem Faserverbund herausfallen können.

Zumindest ein Teil der ersten und/ oder zweiten Fasern des Veloursnadelvliesstoffs könnte als Stapelfasern ausgebildet sein. Stapelfasern können sehr leicht gesplittet werden.

Zumindest ein Teil der ersten und/ oder zweiten Fasern des Veloursnadelvliesstoffs könnte als Elementarfasern ausgestaltet sein, die aus Side-by-side-Fasern, Island-in-the-sea-Fasern und/ oder PIE-Fasem gesplittet sind. Die zuvor genannten Mehrkomponentenfasern können ganz oder teilweise in zwei oder mehrere Elementarfasern aufgesplittet werden. Hierdurch wird die Feinheit der Fasern eines Wundreinigungsprodukts erheblich erhöht.

Zumindest ein Teil der ersten und/ oder zweiten Fasern des Veloursnadelvliesstoffs könnte als Fasern mit einer Feinheit kleiner 1 dtex ausgestaltet sein. Fasern dieser Feinheit werden als Mikrofasern bezeichnet. Durch eine geeignete Wahl des Anteils an Mikrofasern wird die schonende Reinigungswirkung des Veloursnadelvliesstoffs eingestellt. Mikrofasern sind besonders weich und verleihen dem Veloursnadelvliesstoff eine weiche und wundschonende Oberfläche.

Das Splitten von Fasern, insbesondere von Stapelfasern oder von Filamenten in Elementarfasern, um Mikrofasern herzustellen, kann beispielsweise durch eine Kohlendioxid-Behandlung mit Trockeneis bewirkt werden, wie dies beispielsweise in der DE 10 2007 041 630 A1 beschrieben ist.

Es ist jedoch auch denkbar, kommerziell erhältliche Mikrofasern zu verwenden, die nicht mehr gesplittet werden müssen. Vor diesem Hintergrund ist denkbar, eine Mischung aus Mikrofasern und Nicht-Mikrofasern herzustellen und diese zu einem Veloursnadelviiesstoff zu verarbeiten.

Ein hoher Mikrofaseranteil bewirkt eine gründliche Reinigung einer Wunde aufgrund der großen Oberfläche der Mikrofasern. Des Weiteren zeigt ein Wundreinigungsprodukt mit einem hohen Mikrofaseranteil eine erhöhte Feuchtigkeits- und Wasseraufnahmekapazität. Hierdurch wird die Reinigungskraft des Wundreinigungsprodukts durch den Mikrofaseranteil erhöht.

Der Veloursnadelvliesstoff könnte als Tuch, Tasche, Fingerling und/ oder Handschuh konfektioniert sein. Aufgrund der Verwendung mindestens zweier Polymere ist der Faserverlust bei einer Konfektionierung des Veloursnadelvliesstoffs relativ gering. Des Weiteren ist der Faserverlust bei der Reinigung von Wunden relativ gering. Der Veloursnadelvliesstoff lässt sich heiß schneiden oder stanzen. Hierdurch werden Bereiche von Fasern oder einzelne Fasern an den Schnitt- oder Stanzstellen durch Hitze oder Ultraschall versiegelt. So ist keine zusätzliche Versäumung von Rändern notwendig. Hierdurch wird die Keimbelastung gesenkt. Des Weiteren ist kein zusätzlicher Prozessschritt notwendig, um Ränder zu versiegeln. Daher kann ein Wundreinigungsprodukt durch Verwendung eines hier beschriebenen Veloursnadelvliesstoffs besonders ökonomisch gestaltet werden.

Vor diesem Hintergrund ist konkret denkbar, dass der Veloursnadelvliesstoff in eine rechteckige Form und/ oder in eine runde Form verbracht wird. Die Form des Veloursnadelvliesstoffs hängt ganz von der Anwendung des Wundreinigungsprodukts ab. Eine Rechteckform lässt sich besonders gut zuschneiden.

Übliche Produkte zur Wundversorgung weisen eine quadratische Form mit etwa 10 cm Kantenlänge auf.

Sofern der Veloursnadelviiesstoff als Tasche ausgebildet ist, könnte der Veloursnadelvliesstoff zwei Seiten oder auch nur eine Seite der Tasche ausbilden. Sofern der Veloursnadelvliesstoff nur eine Seite der Tasche ausbildet, kann die andere Seite aus einem anderen Werkstoff, insbesondere aus einem Vliesstoff, gefertigt sein. Die Seiten der Tasche könnten unterschiedliche Eigenschaften aufweisen, die auf die jeweilige Anwendung abgestimmt sind.

Die Größe der Tasche kann so ausgelegt werden, dass mehrere Finger in die Tasche hineinpassen. Bei der Reinigung einer Wunde ist die Handhabung einer Tasche einfacher als die eines Tuchs. Die Größe der Tasche kann an die Größe eines Fingers angepasst werden. Insoweit ist die Wundreinigung dosierbar. Ein oder zwei Finger könnten bei kleinen Wunden in eine kleine Tasche gesteckt werden, alle Finger könnten bei großen Wunden in eine größere Tasche gesteckt werden. Insoweit ist eine dosierbare und individuelle Handhabung des Wundreinigungsprodukts möglich.

Dem Veloursnadelvliesstoff könnte eine Griffeinheit zugeordnet sein. Dabei ist konkret denkbar, dass der Veloursnadelvliesstoff auf einer Fläche aufliegt und von oben an einer Griffeinheit ergriffen und mit Druck beaufschlagt werden kann. Hierdurch ist die Nutzung der gesamten Oberfläche eines flächigen Veloursnadelvliesstoffs möglich. Des Weiteren ist dieser problemlos mit Druck beaufschlagbar. Vor diesem Hintergrund ist denkbar, dass die Griffeinheit als Schwamm ausgebildet ist. Des Weiteren ist denkbar, dass die Griffeinheit aus einem Kunststoff besteht.

Die Schlaufen könnten unterschiedlich weit von einer ersten Lage abragen. Diese so genannte unregelmäßige Polhöhe erlaubt eine Anpassung an Haut- und Gewebeschichten. Eine unregelmäßige Polhöhe kann durch eine geeignete Verfahrensführung eingestellt werden. Die Polhöhe, welche sich aus der Schlaufengröße, Tiefe oder Höhe ergibt, kann variabel gestaltet werden.

Bevorzugt werden Lagen aus Stapelfasern verwendet, wobei die Enden der Stapelfasern nicht modifiziert und behandelt sind. Bevorzugt werden kommerziell erhältliche Stapelfasern verwendet, in einem Vliesstoff abgelegt und durch Nadeln in Schlaufen gezwungen. Hierdurch sollen frei auskragende Enden von Fasern vermieden werden.

Die Beweglichkeit der Fasern im Pol ermöglicht eine Anpassung an die Beschaffenheit der Haut- und Gewebeschichten. Durch eine Verringerung des Flächengewichts eines Vliesstoffes und des Anteils der bindenden Fasern im Vliesstoff kann dessen Steifigkeit reduziert werden. So kann sich ein Vliesstoff besser der Kontur eines zu reinigenden Untergrundes anpassen. Mikrofasern passen sich besser Hautstrukturen an, als dickere Nicht-Mikrofasern.

### Kurzbeschreibung der Zeichnung

In der Zeichnung zeigen
- Fig. 1: eine schematische Ansicht eines zweilagigen Wundreinigungsprodukts,
- Fig. 1A: eine schematische Ansicht eines einlagigen Wundreinigungsprodukts und
- Fig. 2: eine teilweise Schnittansicht einer Faser, die in eine Schlaufe gezwungen von einer Lage abragt, wobei die Faser eine kuchenstückförmige Elementarfaser einer PIE4-Mehrkomponentenfaser ist. Ausführung der Erfindung

Verfahren zur Herstellung eines Veloursnadelvliesstoffs entnimmt der Fachmann der WO 2009/039 914 A1.

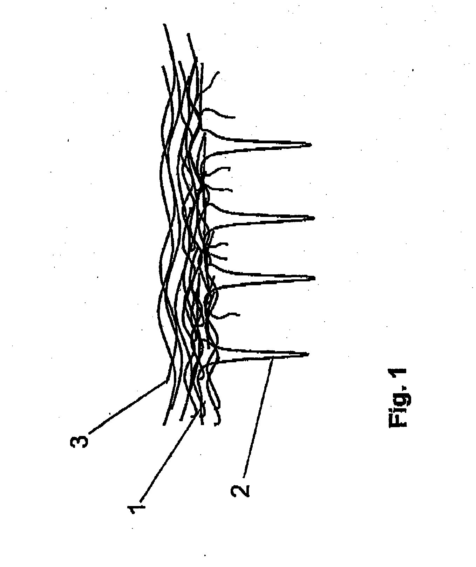

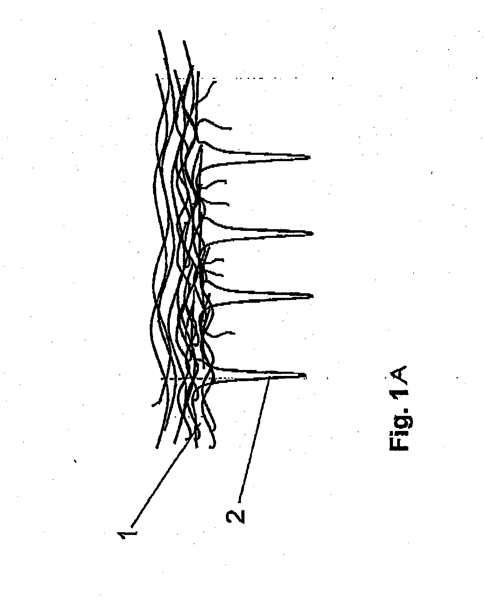

An dieser Stelle soll anhand von Fig. 1 ein bevorzugtes Ausführungsbeispiel eines Veloursnadelvliesstoffs beschrieben werden, welcher zur Herstellung eines Wundreinigungsprodukts besonders geeignet ist.

Ein solcher Veloursnadelvliesstoff weist eine erste Lage 1 mit ersten Fasern auf, welche als Stapelfasern ausgebildet sind. Stapelfasern zeichnen sich durch eine endliche Länge aus. Stapelfasern werden üblicherweise auf eine bestimmte Länge zugeschnitten verwendet. Die Stapelfasern werden in einem zumindest teilweise verfestigten Vliesstoff abgelegt. Darauf wird dieser vorverfestigte Vliesstoff auf einer Stichunterlage abgelegt. Nadeln, welche den Vliesstoff durchstoßen, nehmen Stapelfasern mit und drücken diese als Schlaufen 2 aus dem Vliesstoff heraus.

Auf dieser ersten Lage 1 wird nach der Schlaufenbildung eine zweite Lage 3 abgelegt, welche zweite Fasern umfasst. Die zweite Lage 3 wird auf der Seite der ersten Lage 1 abgelegt, welche den Schlaufen 2 abgewandt ist.

Die zweite Lage 3 ist bevorzugt als Spinnvlies ausgestaltet. Ein Spinnvlies weist Filamente auf, nämlich so genannte Endlosfasern. Im Spinnvlies sind Mehrkomponentenfasern enthalten, welche ein erstes Polymer und ein zweites Polymer aufweisen. Das erste Polymer weist einen Schmelzpunkt auf, der höher liegt als der des zweiten Polymers. Die zweite Lage 3 wird zunächst durch die Nadeln mit der ersten Lage 1 verbunden. Dabei werden die Nadeln dermaßen geführt, dass sie in einer vordefinierten Tiefe in die erste Lage 1 hineindringen. Hierdurch werden die zweiten Fasern, nämlich die Filamente, der zweiten Lage 3 zumindest teilweise mit den ersten Fasern verschlungen.

Darauf wird durch eine thermische Behandlung das zweite Polymer derart angeschmolzen, dass es eine thermische Verbindung mit den ersten Fasern der ersten Lage 1 eingeht.

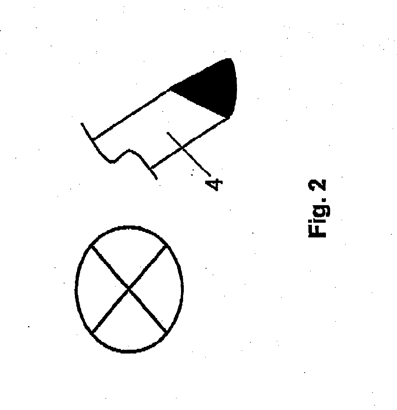

Fig. 2 zeigt im rechten Teil eine teilweise Schnittansicht einer kuchenstückförmigen Elementarfaser 4. Diese Elementarfaser 4 kann in eine Schlaufe gezwungen von einer nicht gezeigten ersten Lage abragen.

Die Elementarfaser 4 kann aus einer PIE4-Mehrkomponentenfaser, wie sie in Fig. 2 im linken Teil im Schnitt dargestellt ist, durch Aufsplitten dieser Mehrkomponentenfaser erzeugt werden.

Nachfolgend wird anhand von zwei Beispielen beschrieben, welche konkreten Fasern und Materialien ein Wundreinigungsprodukt aufweisen kann.

### Beispiel 1:

Für die erste Lage 1 werden Stapelfasern verwendet. Diese Stapelfasern weisen eine Länge von 51 mm und eine Dicke von 2,2 dtex auf. Es handelt sich um Splittfasem der Firma Far Eastern Textile Ltd. Diese Stapelfasern werden zu einem leicht verfestigten Vlies mit einem Flächengewicht von ca. 450 g/m² abgelegt.

Als zweite Lage 3 wird ein Spinnvlies der Far Eastern Textile Ltd. eingesetzt, weiches Monofilamente bestehend aus zwei unterschiedlichen Polyestertypen mit unterschiedlichen Schmelzpunkten enthält. Der Schmelzpunkt der niedrigschmelzenden Komponente liegt bei 190 °C. Das Flächengewicht des Spinnvlieses liegt bei 170 g/m².

In einem Nadelprozess gegen eine Stichunterlage wird die erste Lage 1 so vernadelt, dass die Stapelfasern aus der Vliesebene der ersten Lage 1 heraus gedrückt und zu Schlaufen 2 gelegt werden. Die zweite Lage 3 wird auf die Seite der ersten Lage 1 gelegt, welche den Schlaufen 2 abgewandt ist, und durch einen weiteren Vernadelungsschritt derart vernadelt, dass die Monofilamente der zweiten Lage 3 durch die Stapelfasern der ersten Lage 1 durchgeführt und ebenfalls zu Schlaufen gelegt werden.

Das gesamte entstandene Gebilde wird in einem Heißluftofen bei 200 °C und einer Verweilzeit von 5 Minuten geführt, so dass ein Anschmelzen des niedrigschmelzenden Polyesters stattfindet und dadurch die Fasern beider Lagen 1, 3 mit einander verbunden werden.

Durch das oben beschriebene Nadeln sind die Splittfasern zumindest teilweise in Elementarfasem aufgesplittet worden.

### Beispiel 2:

Als erste Lage 1 wird ein Spinnvlies verwendet. Die verwendeten Filamente weisen eine PIE-Struktur im Querschnitt - analog dem in Fig. 2 links gezeigten Querschnitt, jedoch nicht auf eine Viertelteilung beschränkt - und eine Dicke von 2,2 dtex auf. Das Spinnvlies wurde in einem herkömmlichen Produktionsprozess abgelegt und teilweise leicht verfestigt. Das Flächengewicht beträgt ca. 130 g/m².

Als zweite Lage 3 wird ein Spinnvlies der Far Eastern Textile Ltd. eingesetzt, welches Monofilamente bestehend aus zwei unterschiedlichen Polyestertypen mit unterschiedlichen Schmelzpunkten enthält. Der Schmelzpunkt der niedrigschmelzenden Komponente liegt bei 190 °C. Das Flächengewicht des Spinnvlieses liegt bei 170 g/m².

In einem Nadelprozess gegen eine Stichunterlage wird die erste Lage 1 so vernadelt, dass die Filamente aus der Vliesebene der ersten Lage 1 rausgedrückt und zu Schlaufen 2 gelegt werden. Die zweite Lage 3 wird auf die Seite der ersten Lage 1 gelegt, welche den Schlaufen 2 abgewandt ist, und durch einen weiteren Vernadelungsschritt derart vernadelt, dass die Monofilamente der zweiten Lage 3 durch die Filamente der ersten Lage 1 durchgeführt und ebenfalls zu Schlaufen gelegt werden.

Das gesamte Gebilde wird in einem Heißluftofen bei 200 °C und einer Verweilzeit von 5 Minuten geführt, so dass ein Anschmelzen des niedrigschmelzenden Polyesters stattfindet und dadurch die Fasern beider Lagen 1, 3 mit einander verbunden werden.

Durch das zuvor beschriebene Nadeln sind die Filamente, die im Querschnitt eine PIE-Struktur zeigten, zumindest teilweise in Elementarfasern, nämlich hier in Elementarfilamente, aufgesplittet worden.

In den zuvor genannten Beispielen wird unter einem Monofilament ein Filament, also eine Endlosfaser, verstanden, welche aus einem einzigen Polyester gefertigt ist.

Die beiden Typen der zuvor genannten Monofilamente sind jeweils aus unterschiedlichen Polyestertypen gefertigt. Ein erstes Monofilament ist aus einem ersten Polyester gefertigt und ein zweites Monofilament ist aus einem zweiten Polyester gefertigt, wobei das erste Polyester einen höheren Schmelzpunkt hat als das zweite. Beide Filamente liegen im Spinnvlies nebeneinander vor. Es sind keine Kern-Mantel-Fasern oder ähnliche verwendet worden.

Unter einer Splittfaser wird eine Faser verstanden, die beispielsweise eine PIE-Querschnittsgeometrie - analog zur linken Darstellung gemäß Fig. 2, jedoch nicht auf eine Viertelteilung beschränkt - zeigt. Eine Splittfaser kann durch Zufuhr von Energie, welcher Art auch immer, in Elementarfasem gesplittet werden.

Anstelle der in den Beispielen 1 und 2 verwendeten Spinnvliese könnten auch leicht verfestigte Stapelfaservliese verwendet werden.

## Patentansprüche

1. Verwendung eines Veloursnadelvliesstoffs zum Reinigen von Wunden und/ oder als Wundreinigungsprodukt, welches Gewebe und Körperflüssigkeiten von Wunden und diese umgebender Haut in sich oder an sich aufnimmt, wobei der Veloursnadelvliesstoff in Schlaufen (2) gezwungene Fasern umfasst, wobei der Veloursnadelvliesstoff ein erstes Polymer und ein zweites Polymer umfasst und wobei das erste Polymer einen höheren Schmelzpunkt hat als das zweite Polymer.

2. Verwendung nach Anspruch 1, **dadurch** gekennzeichet, **dass** der Veloursnadelvliesstoff durch Ablage eines zumindest teilweise vorverfestigten Vliesstoffs auf einer Stichunterlage und durch Nadeln des Vliesstoffs auf dieser Stichunterlage hergestellt ist.

3. Verwendung nach Anspruch 1 oder 2, **dadurch gekennzeichnet, dass** der Veloursnadelvliesstoff nur eine Lage (1) aufweist, von welcher Schlaufen (2) abragen.

4. Verwendung nach Anspruch 1 oder 2, **dadurch gekennzeichnet, dass** der Veloursnadelvliesstoff eine erste Lage (1) und eine zweite Lage (3) aufweist, wobei die erste Lage (1) erste Fasern umfasst, die in Schlaufen (2) gezwungen von der ersten Lage (1) abragen, und wobei die zweite Lage (3) zweite Fasern umfasst, welche mit der ersten Lage (1) zumindest bereichsweise verbunden sind.

5. Verwendung nach Anspruch 4, **dadurch gekennzeichnet, dass** auch die zweiten Fasern in Schlaufen gezwungen sind.

6. Verwendung nach einem der voranstehenden Ansprüche, **dadurch gekennzeichnet, dass** der Veloursnadelvliesstoff erste und/ oder zweite Fasern umfasst, die als Mehrkomponentenfasern ausgebildet sind, welche das erste und das zweite Polymer aufweisen.

7. Verwendung nach einem der voranstehenden Ansprüche, **dadurch gekennzeichnet, dass** der Veloursnadelvliesstoff als erste und/ oder zweite Fasern strukturbildende Fasern mit dem ersten Polymer und als erste und/ oder zweite Fasern bindende Fasern mit dem zweiten Polymer umfasst.

8. Verwendung nach einem der voranstehenden Ansprüche, **dadurch gekennzeichnet, dass** zumindest ein Teil der ersten und/ oder zweiten Fasern des Veloursnadelvliesstoffs als Filamente ausgebildet ist.

9. Verwendung nach einem der voranstehenden Ansprüche, **dadurch gekennzeichnet, dass** zumindest ein Teil der ersten und/ oder zweiten Fasern des Veloursnadelvliesstoffs als Elementarfasern ausgestaltet ist, die aus Side-by-side-Fasern, Island-in-the-sea-Fasern und/ oder PIE-Fasern gesplittet sind.

10. Verwendung nach einem der voranstehenden Ansprüche, **dadurch gekennzeichnet, dass** zumindest ein Teil der ersten und/ oder zweiten Fasern des Veloursnadelvliesstoffs als Fasern mit einer Feinheit kleiner 1 dtex ausgestaltet ist.

11. Verwendung nach einem der voranstehenden Ansprüche, **dadurch gekennzeichnet, dass** der Veloursnadelvliesstoff als Tuch, Tasche, Fingerling und/ oder Handschuh konfektioniert ist.

12. Verwendung nach einem der voranstehenden Ansprüche, **dadurch gekennzeichnet, dass** dem Veloursnadelviiesstoff eine Griffeinheit zugeordnet ist.

## Claims

1. Use of a velour-type needlepunched nonwoven fabric for the cleaning of wounds and/or as a wound-cleaning product which ad- or absorbs tissue and bodily fluids from wounds and their surrounding skin, wherein the velour-type needlepunched nonwoven fabric comprises fibres forced into loops (2), wherein the velour-type needlepunched nonwoven fabric comprises a first polymer and a second polymer, and wherein the first polymer has a higher melting point than the second polymer.

2. Use according to Claim 1, **characterized in that** the velour-type needlepunched nonwoven fabric is obtained by laying an at least partially preconsolidated nonwoven fabric down on a bedplate and by needlepunching the nonwoven fabric on this bedplate.

3. Use according to Claim 1 or 2, **characterized in that** the velour-type needlepunched nonwoven fabric has only one ply (1) wherefrom loops (2) protrude.

4. Use according to Claim 1 or 2, **characterized in that** the velour-type needlepunched nonwoven fabric has a first ply (1) and a second ply (3), wherein the first ply (1) comprises first fibres which forced into loops (2) protrude from the first ply (1), and wherein the second ply (3) comprises second fibres which are at least regionally connected to the first ply (1).

5. Use according to Claim 4, **characterized in that** the second fibres are also forced into loops.

6. Use according to any preceding claim, **characterized in that** the velour-type needlepunched nonwoven fabric comprises first and/or second fibres formed as multicomponent fibres which include the first and second polymers.

7. Use according to any preceding claim, **characterized in that** the velour-type needlepunched nonwoven fabric comprises, as first and/or second fibres, structure-forming fibres comprising the first polymer and, as first and/or second fibres, binding fibres comprising the second polymer.

8. Use according to any preceding claim, **characterized in that** at least some of the first and/or second fibres of the velour-type needlepunched nonwoven fabric are formed as filaments.

9. Use according to any preceding claim, **characterized in that** at least some of the first and/or second fibres of the velour-type needlepunched nonwoven fabric are formed as elementary fibres split from side-by-side fibres, island-in-the-sea fibres and/or PIE fibres.

10. Use according to any preceding claim, **characterized in that** at least some of the first and/or second fibres of the velour-type needlepunched nonwoven fabric are formed as fibres having a fineness below 1 dtex.

11. Use according to any preceding claim, **characterized in that** the velour-type needlepunched nonwoven fabric is made up as cloth, bag, fingerstall and/or glove.

12. Use according to any preceding claim, **characterized in that** the velour-type needlepunched nonwoven fabric is assigned a grip unit.

## Revendications

1. Utilisation d'un non-tissé aiguilleté en velours pour le nettoyage de lésions et/ou en tant que produit de nettoyage de lésions, qui absorbe ou adsorbe les tissus et les fluides corporels issus de lésions et de la peau environnante, le non-tissé aiguilleté en velours comprenant des fibres agencées en boucles (2), le non-tissé aiguilleté en velours comprenant un premier polymère et un second polymère, et le premier polymère présentant un point de fusion plus élevé que le second polymère.

2. Utilisation selon la revendication 1, **caractérisée en ce que** le non-tissé aiguilleté en velours est fabriqué par dépôt d'un non-tissé au moins partiellement pré-consolidé sur une sous-couche de piqûre, et par aiguilletage du non-tissé sur cette sous-couche de piqûre.

3. Utilisation selon la revendication 1 ou 2, **caractérisée en ce que** le non-tissé aiguilleté en velours ne comprend qu'une couche (1), de laquelle des boucles (2) dépassent.

4. Utilisation selon la revendication 1 ou 2, **caractérisée en ce que** le non-tissé aiguilleté en velours comprend une première couche (1) et une seconde couche (3), la première couche (1) comprenant des premières fibres qui dépassent de la première couche (1) agencées en boucles (2), et la seconde couche (3) comprenant des secondes fibres, qui sont reliées au moins par zones avec la première couche (1).

5. Utilisation selon la revendication 4, **caractérisée en ce que** les secondes fibres sont également agencées en boucles.

6. Utilisation selon l'une quelconque des revendications précédentes, **caractérisé en ce que** le non-tissé aiguilleté en velours comprend des premières et/ou des secondes fibres qui sont configurées sous la forme de fibres multicomposantes, qui comprennent le premier et le second polymère.

7. Utilisation selon l'une quelconque des revendications précédentes, **caractérisée en ce que** le non-tissé aiguilleté en velours comprend en tant que premières et/ou secondes fibres des fibres structurantes comprenant le premier polymère et en tant que premières et/ou secondes fibres des fibres liantes comprenant le second polymère.

8. Utilisation selon l'une quelconque des revendications précédentes, **caractérisée en ce qu'**au moins une partie des premières et/ou secondes fibres du non-tissé aiguilleté en velours sont configurées sous la forme de filaments.

9. Utilisation selon l'une quelconque des revendications précédentes, **caractérisée en ce qu'**au moins une partie des premières et/ou secondes fibres du non-tissé aiguilleté en velours sont configurées sous la forme de fibres élémentaires, qui sont divisées à partir de fibres side-by-side, de fibres island-in-the-sea et/ou de fibres PIE.

10. Utilisation selon l'une quelconque des revendications précédentes, **caractérisée en ce qu'**au moins une partie des premières et/ou secondes fibres du non-tissé aiguilleté en velours sont configurées sous la forme de fibres d'une finesse inférieure à 1 dtex.

11. Utilisation selon l'une quelconque des revendications précédentes, **caractérisée en ce que** le non-tissé aiguilleté en velours est confectionné sous la forme d'un drap, d'une poche, d'un doigtier et/ou d'un gant.

12. Utilisation selon l'une quelconque des revendications précédentes, **caractérisée en ce que** le non-tissé aiguilleté en velours est attribué à une unité de poignée.
